# EUROPÄISCHE PATENTSCHRIFT

(11) **EP 2 156 933 B1**
(45) Veröffentlichungstag und Bekanntmachung des Hinweises auf die Patenterteilung: **25.12.2013**
(21) Anmeldenummer: 09162044.3
(22) Anmeldetag: 05.06.2009
(51) Int. Cl.: B29B 9/06, C08G 69/14, C08G 69/16, C08G 69/46, B01D 11/02, C08J 3/12, C07D 201/16

(54) **Kontinuierliches Verfahren zur Extraktion von Polyamid-6**
Continuous method for extracting polyamide 6
Procédé continu d'extraction de polyamide 6

(30) Priorität: 19.08.2008 DE 102008044452
(43) Veröffentlichungstag der Anmeldung: 24.02.2010
(73) Patentinhaber: Lurgi Zimmer GmbH, 60295 Frankfurt am Main (DE)
(72) Erfinder: KIRSTEN, Klaus, 55130, Mainz (DE); ALBRECHT, Manfred, 61486, Bruchköbel (DE); SAMLITSCHKA, Franz, 63477, Maintal (DE)
(74) Vertreter: Meyer-Dulheuer, Karl-Hermann

(56) Entgegenhaltungen:
- WO-A-2009/067995
- DD-A5- 282 618
- FRANZ FOURNÉ: "Synthetic fibers; Machines and Equipment; Manufacture; Properties" 1999, HANSER PUBLISHERS , MUNICH , XP002562892 * Seiten 33-55, insbesondere Seite 54 und Seiten 242-253, insbesondere Seite 249 *
- Y.P. Wang ET AL: "Wastewater minimisation", Chemical Engineering Science, vol. 49, no. 7, 1 April 1994 (1994-04-01), pages 981-1006, XP055052566, ISSN: 0009-2509, DOI: 10.1016/0009-2509(94)80006-5

## Beschreibung

Gegenstand der Erfindung ist ein kontinuierliches Verfahren zur Einsparung von Energie- und Rohstoffkosten bei der Extraktion von monomerem Caprolactam und daraus entstandenen Oligomeren aus dem bei der Polymerisation zu Polyamid-6 erhaltenen Polymer-Rohprodukt.

Die bei der Herstellung von Polyamiden durch Polymerisation von ε-Caprolactam entstehenden Polymere enthalten niedermolekulare Anteile, die aus Caprolactam und deren Oligomeren bestehen. In der Praxis werden diese niedermolekularen Anteile durch Extraktion mit heißem Wasser entfernt. Aus diesen Extraktionswässern kann man den Caprolactam-Anteil zurückgewinnen, reinigen und ggf. wieder in die Polymerisation einführen. Es ist auch möglich durch Zusatz von spaltenden Reagenzien die in den Extraktwässern enthaltenen Oligomere zu Caprolactam umzusetzen und dieses dann zu isolieren, zu reinigen und wieder zu verwenden.

Die meisten bekannten Verfahren haben den Nachteil, dass eine zum Teil mehrstufige Aufarbeitung des Extraktwassers erfolgen muss, bevor der gesamte Extrakt oder Extraktbestandteile, insbesondere ε-Caprolactam, zur erneuten Polymerisation eingesetzt werden können. Die Verfahren, die eine Abtrennung, Aufarbeitung und Recyclisierung von Caprolactam vorschlagen, weisen zudem den Nachteil auf, dass die in den Extraktwässern enthaltenen Oligomere oft nicht aufgearbeitet werden, sondern entsorgt werden müssen. Des Weiteren wird in den genannten Verfahren zur Wiederverwertung von Extraktwasser der Einsatz einer Verfahrensstufe zur hydrolytischen Polymerisation des Extraktwasserskonzentrats oder einer Mischung aus Extraktwasserbestandteilen und Caprolactam vorausgesetzt.

Das durch Polymerisation zu Polyamid-6 erhaltene Polymer-Rohprodukt enthält je nach Temperatur im Gleichgewicht 8 - 15 Gew.-% Caprolactam und dessen Oligomere. Diese stören die weitere Verarbeitung und werden daher meist nach der Granulierung durch Extraktion mit heißem Wasser aus der Polymermatrix herausgelöst. Das dabei verwendete Wasser, das Extraktwasser, wird aus wirtschaftlichen Gründen in einer mehrstufigen Destillationsanlage ("Rückgewinnungsanlage") eingedampft und der Rückstand erneut als Rohstoff der Polymerisation zugeführt.

Es besteht jedoch generell das Ziel, diese Wassermenge so gering wie möglich zu halten, denn Wasser zu verdampfen kostet Energie.

Bei den bekannten Verfahren wird das Polyamid-6-Polymer in Unterwassergranulatoren (UWG) oder in Unterwasserstranggranulatoren (USG) zu Zylindern oder Kugeln geschnitten. Beide Arten von Granulatoren benötigen kühlende Flüssigkeiten, um die Polymerschmelze zum Erstarren zu bringen und anschließend die Polymerpartikel abzukühlen. Diese Kühlflüssigkeit ist normalerweise Wasser, das im nahezu geschlossenen Kreislauf zirkuliert wird.

Dieses Wasser reichert sich im Laufe des Verfahrens mit Caprolactam und Oligomeren aus dem Polymer an, so dass dem im Kreis geführten Wasser von Zeit zu Zeit sauberes Wasser zugemischt und es damit aufgefrischt werden muss. Außerdem müssen auch Verdampfungs- und Leckageverluste ausgeglichen werden.

Den Granulatorsystemen wird sauberes, meistens entsalztes, gereinigtes Wasser zugespeist. Das ausgeschleuste Wasser aus den Granulatorsystemen wird entweder verworfen oder der Rückgewinnungsanlage zugeführt, um Caprolactam und die Oligomeren zurück zu gewinnen. Die Verwendung des Extraktwassers hängt im Einzelfall von dem Extraktgehalt, dem Preis des Caprolactams und den Energiekosten ab. In manchen Fällen ist es wirtschaftlicher, dass Caprolactam zu verwerfen, als das Wasser einzudampfen.

Bei den vorstehend genannten Verfahren hat sich technisch die kontinuierliche oder auch diskontinuierliche Extraktion von PA-6-Chips mit heißem Wasser durchgesetzt. Mit diesem Verfahren werden Monomer- und Oligomergehalte von < 0,5 Gew.-% im PA-6-Chip erreicht. Derartig niedrige Monomer- und Oligomergehalte im Polymeren sind erforderlich, wenn das Polyamid für Spinnereizwecke verwendet werden sollen.

Aus Gründen der Wirtschaftlichkeit werden die wässrigen Extraktlösungen so aufbereitet, dass die darin enthaltenen Wertstoffe als Rohstoff dem Polycaprolactam-Herstellungsprozess zugeführt werden können. Nach einer einfachen Einengung des Extraktwassers durch Verdampfen des Wassers verbleiben neben dem monomeren γ-Caprolactam auch die cyclischen Dimeren und weitere Oligomere im Rückcaprolactam.

In der DE 2501348 B1 wird die Einengung des Extraktwassers auf mehr als 90 Gew.-% Extraktanteil mit anschließender direkter Zuführung in die Polymerisationsstufe mit und ohne Frischcaprolactamzusatz beschrieben. Gemäß EP 0000397 B1 kann auch Extraktwasser in die Polymerisation zurückgeführt werden, das auf maximal 60 Gew.-% Extraktanteil eingeengt wurde. In beiden Fällen werden die Extraktlösungen - mit oder ohne Frischcaprolactam-Zusatz - vor der Zudosierung in den VK-Rohrkopf temperiert, damit das hoch schmelzende und schwerlösliche cyclische Dimere des ε-Caprolactams unter diesen Bedingungen in Lösung bleibt, sodass keine Leitungsverstopfungen und dergleichen auftreten. Die für den nachfolgenden Einbau in die Polymerkette notwendige Spaltung des cyclischen Dimeren kann aber auf diesem Wege nicht ausreichend sichergestellt werden.

EP 0771834 A1 beschreibt die Einengung des Extraktwassers mit anschließender partieller Ringöffnungsreaktion der Oligomeren zu linearen kondensierbaren Verbindungen unter Reaktionsbedingungen von 230°C - 300°C bei definierten Drucken, die bis zu 10 h gehalten werden. Die so behandelten Extrakte werden anschließend zusammen mit Frischcaprolactam in einem Reaktor polymerisiert, wobei teilweise Wasserkonzentrationen von bis zu 10 Gew.-% enthalten sein können. In US 5218080 A wird die hydrolytische Dimerenspaltung des konzentrierten Extraktes unter Druck bei 200 - 290° C während 2 - 6 h durchgeführt, wobei der so erhaltene Extrakt mit ca. 1,3 Gew.-% Dimeren direkt dem Frischlactam in Mengen bis zu 10 Gew.-% zugesetzt wird. Vor dem Hintergrund zunehmender Kapazitätsvergrößerungen kontinuierlich betriebener hydrolytischer Caprolactam-Polymerisationsanlagen ist die Wirtschaftlichkeit dieser Verfahren bzw. die Höhe des noch verbleibenden Dimerengehaltes im so aufbereiteten Extrakt aber verbesserungsbedürftig.

Des Weiteren ist ein Prozess bekannt, in dem das Extraktwasser nach einer Aufkonzentration auf ca. 80 Gew.-% γ-Caprolactam/Oligomere ohne weitere Frischcaprolactam-Zuführung in einer zweiten, separaten Polymerisationslinie zu PA-6 polymerisiert wird (Chemical Fibres International 47, 316 (1997)). Nachteil dieses Verfahrens sind die hohen Investitionskosten für die komplette zweite Polymerisationslinie, in der eine Dimeren-Reaktivierung unter Polymerisationsbedingungen erfolgt, die von denen des Frischcaprolactam-Polymerisationsprozesses der ersten Linie abweichen. Der erhöhte Wasseranteil verschlechtert die Ökonomie dieser 2. Linie.

Andere Verfahren, die eine separate Aufbereitung der im Extraktwasser anfallenden Oligomeren und cyclischen Dimeren zum Inhalt haben, setzen eine Trennung dieser Bestandteile vom Extraktwasser voraus. US 5653889 A beschreibt eine Filtrationstechnik zur Separierung der Oligomeren aus dem Prozesswasser der PA-6-Granulierungsstude. Diese Filtrationtechnik ist auf eine Oligomer-Separierung und Aufbereitung einer bis zu 15 Gew.-%igen wässrigen Extraktlösung aus der Polymerisation, die auch monomeres ε-Caprolactam enthält, nicht ohne weiteres übertragbar.

Zur Aufbereitung der Oligomeren kann ein Verfahren gemäß US 4107160 A zur Anwendung kommen, indem - neben PA-6-Feststoffabfällen - die Oligomeren in Gegenwart eines Katalysators und überhitztem Wasserdampf depolymerisiert werden. Nach anschließender Einengung lässt sich eine ca. 50 Gew.-%ige wässrige ε-Caprolactamlösung gewinnen, die dann gemäß DE 4316408 A1 nach einer Raffinationsstufe mit Permanganaten und einer Aktivkohlebehandlung filtriert und eingedampft wird; nach einer Feindestillation kann das gewonnene Reincaprolactam in einen PA-6 Herstellungsprozess zurückgeführt werden . Dieses aufwendige Verfahren, das eine hohe Rückcaprolactamqualität liefert, wird durch zahlreiche Verfahrensstufen mit entsprechend hohem Energieaufwand und durch Verbrauchsmittel, wie Permanganat und Aktivkohle, von erhöhtem Kostenaufwand begleitet.

Die alternativ mögliche Verwerfung und Entsorgung der aus dem Extraktwasser isolierten Oligomeren reduziert die Rohstoffausbeute deutlich und stellt somit keine ökonomische Verfahrensweise dar, insbesondere bei steigenden Anlagenkapazitäten.

Ferner wir in der GB 1,297,263A die Verwendung einer Katalysators zur Depolymerisation der Oligomeren erwähnt. Als ein möglicher Katalysator wird Phosphorsäure genannt. Was mit dem Depolymerisationsprodukt geschieht, wird jedoch dort nicht beschrieben. Insbesondere erwähnt die genannte britische Patentschrift nicht die zusätzliche Einspeisung von überhitztem Wasserdampf in diese Spaltungsstufe.

In der DE-A-43 21 683 und in der US 4.049.638 sind Verfahren zur Polycaprolactamherstellung beschrieben, die den Einsatz von Caprolactam mit bis zu 15 % Wassergehalt in der Polymerisation erlauben. Die EP-A-0 745 631 offenbart die Wiederverwendung von wässrigen Extraktlösungen unter Zusatz geringer Mengen einer Di- oder Polycarbonsäure, da sonst der Extrakt langsamer polymerisiert als Caprolactam.

Da der Extrakt auch erhebliche Anteile an cyclischen Oligomeren enthält, die bei der Polymerisation unverändert bleiben, wurden verschiedene Verfahren zur Spaltung dieser Oligomere beziehungsweise Überführung in lineare Oligomere vorgeschlagen. Die Oligomere werden üblicherweise mit Phosphorsäure oder durch Einsatz hoher Temperaturen gespalten. So beschreibt die US 5,077. 381 ein Verfahren zur Spaltung der Oligomere bei Temperaturen von 220° bis 290° C, vorzugsweise unter erhöhten Druck.

Vor der Rückführung in die Polymerisation muss die üblicherweise etwa 10 Gew.-%ige Extraktlösung zunächst aufgearbeitet d.h. in der Regel aufkonzentriert werden. Die Aufarbeitung erfolgt normalerweise durch Abdestillieren des Wassers. Die DE-A-25 01 348 beschreibt die Aufkonzentrierung unter Ausschluss von Luftsauerstoff, wobei vor der Aufkonzentrierung auf mehr als 70 Gew.-% dem Extraktwasser frisches Caprolactam zugesetzt wird, wodurch ein Ausfallen von Oligomeren vermindert wird.

Bei der Anwendung der oben genannten Verfahren zur Extraktwasserrückführung ergibt sich jedoch ein schwerwiegender Nachteil: Die kontinuierliche Rückführung des Extraktwassers bedingt einen starken Konzentrationsanstieg der Oligomere und der thermodynamisch stabilen cyclischen Dimere nicht nur im Reaktionsgemisch, sondern auch im Polymer, wenn im Zuge der kontinuierlichen hydrolytischen Lactampolymerisation die Aufspaltung der Oligomere nicht gelingt beziehungsweise die Einstellung der chemischen Gleichgewichte zu langsam ist. Zudem ist der Anstieg der Oligomerkonzentration dann besonders hoch, wenn das Reaktionsgemisch - beispielsweise zur Herstellung von Polyamiden mit hohem Molekulargewicht - einen geringen Wassergehalt aufweist.

Allen bisher bekannten Verfahren ist gemeinsam, dass große Mengen Extraktionswasser, enthaltend monomeres Caprolactam und das daraus entstandene Oligomere, anfallen, die einer nachfolgenden Polyamidsynthese zugeführt werden müssen, weil es sich um wertvolle chemische Rohstoffe handelt. Dazu muss die wässrige Extraktionslösung eingedampft werden, was mit hohen Energiekosten verbunden ist.

Die WO 2009/067995 betrifft ein Verfahren zur Herstellung von Granulatkörnern von Polyamid 6 oder von Copolyamiden, wobei die Unterwassergranulation und die Extraktion unter Verwendung desselben Prozessfluids erfolgen und das Prozessfluid eine Zusammensetzung aufweist, welche einer Zusammensetzung nach der Extraktion entspricht, wobei das Prozessfluid bevorzugt mehr als 10 Gew.-% Extrakt aufweist. Die DD 282 618 A5 beschreibt ein Verfahren zur Abkühlunq von schmelzflüssigen Polyamidbändern und -drähten, wobei als Kühlmedium ein extrakthaltipes Wasser verwendet wird, welches mit 8 bis 12 % niedermolekularen Anteilen aus der Extraktion des Granulats angereichert ist. Bei den beiden letztgenannten Verfahren werden jedoch mögliche Beeinträchtigungen des Granulationsprozess nicht berücksichtigt.

Es stellte sich deshalb die Aufgabe, Einsparungen von Energie- und Rohstoffkosten bei der Extraktion von monomerem Caprolactam und dem daraus entstandenen Oligomeren aufzufinden. Um die Menge de einzudampfenden Wassers zu minimieren, wird der Umstand ausgenutzt, dass die Granulation ohnehin mit extrakthaltigem Wasser betrieben wird.

Gegenstand der Erfindung ist ein Verfahren gemäß Anspruch 1.

Die erfindungsgemäßen Prozessschritte sind wie folgt:
- Entnahme von Extraktwasser aus dem unteren Bereich der Extraktionskolonne, mit einem Extraktgehalt von ca. 1 bis 2 %.
- Verwendung dieses Extraktwassers als Prozesswasser für die Granulation bis der Extraktgehalt auf ca. 4 bis 6 % gestiegen ist. Höhere Extraktgehalte des Granulator-Prozesswassers können zu Störungen des Granulationsprozeses führen.
- Rückführung des angereicherten Prozesswassers aus der Granulation in die Extraktionskolonne. Dort wird es dem Prozesswasser der Extraktionskolonne untergemischt und bis zu Extraktkonzentration von ca. 10 % weiterverwendet.

Anstatt sauberes Wasser zuzuspeisen, wird erfindungsgemäß "Extraktwasser" mit einem geringen Extraktgehalt, nämlich weniger als 1 Gew.-% und höchstens 15 Gew.-% an Monomerem oder Oligomeren, vorzugsweise höchstens 10 Gew.-% an Monomerem oder Oligomeren, eingesetzt.

Das ausgeschleuste Wasser wird dann aus dem Granulatorsystem in die Extraktionsanlage oder direkt in die Rückgewinnungsanlage geführt, wo der enthaltene Extrakt zurück gewonnen wird. Dabei werden das Wasser und das Monomere und die Oligomeren voneinander getrennt.

Der Vorteil dieses Verfahrens besteht darin, dass kein Frischwasser für das Granulatorsystem benötigt wird. Daraus ergibt sich ein geringerer Energieverbrauch und ein geringerer Rohstoffverbrauch bei der Polyamid-6-Herstellung, je nach dem, ob das Wasser aus dem Granulatorsystem eingedampft oder verworfen wird.

Die bei der Polyamidextraktion anfallenden Extraktwässer besitzen im allgemeinen einen Gehalt an organischen und gegebenenfalls anorganischen Bestandteilen von 4 bis 15 Gew.-%. Um in die Polymerisation zurückgeführt werden zu können, müssen diese Extraktwässer zunächst eingedampft werden. Dies erfolgt in an sich bekannter Weise in einer ein- oder mehrstufigen Eindampfanlage mit kurzer Verweilzeit, beispielsweise in einem Robertverdampfer, Fallfilmverdampfer, Dünnschichtverdampfer oder Umlaufverdampfer. Das Eindampfen erfolgt bis zu einem Extraktgehalt von höchstens 85 Gew.-%, weil bei dieser Konzentration noch keine Ausfällungen der gelösten Bestandteile zu beobachten sind. Vorzugsweise dampft man auf einen Extraktgehalt von 60 bis 85 Gew.-%, insbesondere 70 bis 85 Gew.-%, ein. Die Eindampftemperaturen liegen dabei im Allgemeinen im Bereich von 103°C bis 115°C, vorzugsweise 107°C bis 112°C (bei Normaldruck). Im Allgemeinen wird das Eindampfen kontinuierlich durchgeführt.

### Vergleichsbeispiel 1 - Stand der Technik

Aus einem Polymerisationsbehälter werden pro Stunde 5.500 kg Polyamid 6 als Polymerschmelze in einen Stranggranulator eingeleitet. Dort werden pro Stunde 900 kg Frischwasser zugesetzt und extrakthaltiges Wasser, bei dem sich die Aufarbeitung nicht lohnt, abgezogen und verworfen.

Durch das umlaufende Wasser findet dort bereits eine Vor-Extraktion des Polyamids statt, wobei sich der Extraktgehalt der im Kreislauf befindlichen wässrigen Caprolactam- und Oligomerenlösung kontinuierlich erhöht.

Das in dem Stranggranulator gewonnene Rohgranulat wird dann in den Extraktor eingeführt, mit 5.500 kg pro Stunde Frischwasser behandelt und dabei ein Extraktwasser mit einem Extraktgehalt von 10 %, besteht aus Caprolactam und Oligomeren, abgelassen. Aus dem Extraktor wird dann ein feuchtes Polymerprodukt entnommen, das fast frei von Extrakt ist, aber noch etwa 10% Wasser enthält. In Summe werden bei den beiden Systemen Granulation und Extraktion eine gesamte Menge von 6.400 kg pro Stunde an Frischwasser zugeführt.

### Vergleichsbeispiel 2 - Stand der Technik

Aus einem Polymerisationsbehälter werden pro Stunde 5.500 kg Polyamid 6 als Polymerschmelze in einen Unterwassergranulator eingeführt. In den Unterwassergranulator werden pro Stunde 550 kg Frischwasser eingeführt und eine entsprechende Menge Extraktwasser abgezogen.

Durch das umlaufende Wasser findet dort bereits eine Vor-Extraktion des Polyamids statt, wobei sich der Extraktgehalt der im Kreislauf befindlichen wässrigen Caprolactam- und Oligomerenlösung kontinuierlich erhöht.

Aus dem Unterwassergranulator wird das Rohgranulat in den Extraktor überführt, dort mit 5.500 kg pro Stunde Frischwasser behandelt und eine entsprechende Menge Caprolactam und Oligomere enthaltendes Extraktwasser abgezogen. Das aus dem Unterwassergranulator gewonnene Extraktwasser und das aus dem Extraktor gewonnene Extraktwasser werden vereinigt, eingedampft und der enthaltene Extrakt zurück gewonnen und wieder in den Polymerisator eingeführt. Es werden pro Stunde 595 kg Extrakt zurück gewonnen. Aus dem Extraktor wird ein feuchtes Produkt entnommen, das fast extraktfrei ist, aber noch etwa 10 % Wasser enthält.

In Summe werden bei den beiden Systemen Granulation und Extraktion eine gesamte Menge von 6.050 kg pro Stunde an Frischwasser zugeführt.

### Beispiel 3: Anwendung der Erfindung unter Verwendung eines Stranggranulators

Aus einem Polymerisationsbehälter werden pro Stunde 5.500 kg Polyamid 6 als Polymerschmelze in einen Stranggranulator geleitet.

Durch das umlaufende Wasser findet dort bereits eine Vor-Extraktion des Polyamids statt, wobei sich der Extraktgehalt der im Kreislauf befindlichen wässrigen Caprolactam- und Oligomerenlösung kontinuierlich erhöht.

Das in dem Stranggranulator gewonnene Rohgranulat wird dann in den Extraktor eingeführt und mit 5.500 kg pro Stunde Frischwasser behandelt.

Im Gegenstrom zu dem zu extrahierenden Polyamidgranulat reichert sich das Wasser mit Monomeren und Oligomeren der Polyamidherstellung an, welche aus dem Polyamidgranulat herausgelöst werden. Das Extraktwasser erreicht am Ende des Extrahiervorgangs eine Konzentration an Caprolactam und Oligomeren von etwa 10 %.

Nachdem das Wasser eine gewisse Strecke im Extraktionsrohr zurückgelegt hat und eine Konzentration von ca. 0,2 % Extrakt erreicht hat, wird erfindungsgemäß ein Teilstrom von 1.200 kg pro Stunde mittels geeigneter Vorrichtung (Sieb, Filter etc.) aus dem Extraktionsrohr ausgeschleust. Dieses extrakthaltige Wasser wird kontinuierlich zum Granulatorsystem geleitet, wo es sich mit dem dort im Umlauf befindlichen Wasser vermischt und dessen Extraktkonzentration senkt (verdünnt).

Die Füllmenge des Granulatorsystems wird konstant geregelt, und das überschüssige Wasser, das als o. g. Teilstrom zugeführt wurde, aber nicht benötigt wird um unwesentliche Verdunstungs- und andere Verluste auszugleichen, wird aus dem Granulatorkreislauf zurück in das Extraktionsrohr geleitet.

Durch die Anreicherung an Extrakt im Granulatorsystem und der Verdünnung durch die zugespeiste Teilmenge beträgt der Extraktgehalt dieses zurückgeleiteten Wassers etwa 0,7 %.

Die Einspeisestelle dieses Wassers in das Extraktionsrohr befindet sich oberhalb der Entnahmestelle und idealerweise auf einer Höhe, wo die Extraktkonzentrationen des zurückgespeisten und des im Reaktor verbliebenen Wassers gleich sind.

Erfindungsgemäß wird den beiden verbundenen Systemen Granulation und Extraktion eine gesamte Menge von 5.500 kg pro Stunde Frischwasser oder wässrige Kondensate zugespeist.

Aus dem Extraktor wird ein feuchtes Produkt entnommen, das fast extraktfrei ist und noch ca. 10 % Wasser enthält.

### Vergleichsbeispiel 4:

Aus einem Polymerisationsbehälter werden pro Stunde 5.500 kg Polyamid 6 als Polymerschmelze in einen Unterwassergranulator geleitet.

Durch das umlaufende Wasser findet dort bereits eine Vor-Extraktion des Polyamids statt, wobei sich der Extraktgehalt der im Kreislauf befindlichen wässrigen Caprolactam- und.Oligomerenlösung kontinuierlich erhöht.

Das in dem Unterwassergranulator gewonnene Rohgranulat wird dann in den Extraktor eingeführt und mit 5.500 kg pro Stunde Frischwasser behandelt.

Im Gegenstrom zu dem zu extrahierenden Polyamidgranulat reichert sich das Wasser mit Monomeren und Oligomeren der Polyamidherstellung an, welche aus dem Polyamidgranulat herausgelöst wurden. Das Extraktwasser erreicht am Ende des Extrahiervorgangs eine Konzentration an Caprolactam und Oligomeren von etwa 10 %.

Nachdem das Wasser eine gewisse Strecke im Extraktionsrohr zurückgelegt hat und eine Konzentration von ca. 0,3 % Extrakt erreicht hat, wird erfindungsgemäß ein Teilstrom von 580 kg pro Stunde mittels geeigneter Vorrichtung (Sieb, Filter, etc.) aus dem Extraktionsrohr ausgeschleust. Dieses extrakthaltige Wasser wird kontinuierlich zum Granulatorsystem geleitet, wo es sich mit dem dort im Umlauf befindlichen Wasser vermischt und dessen Extraktkonzentration senkt (verdünnt).

Die Füllmenge des Granulatorsystems wird konstant geregelt, und das überschüssige Wasser, das als o. g. Teilstrom zugeführt wurde, aber nicht benötigt wird um unwesentliche Verdunstungs- und andere Verluste auszugleichen, wird aus dem Granulatorkreislauf zurück in das Extraktionsrohr geleitet.

Durch die Anreicherung an Extrakt im Granulatorsystem und der Verdünnung durch die zugespeiste Teilmenge beträgt der Extraktgehalt dieses zurückgeleiteten Wassers etwa 6,2 %.

Die Einspeisestelle dieses Wassers in das Extraktionsrohr befindet sich oberhalb der Entnahmestelle und idealerweise auf einer Höhe, wo die Extraktkonzentrationen des zurückgespeisten und des im Reaktor verbliebenen Wassers gleich sind.

Erfindungsgemäß wird den beiden verbundenen Systemen Granulation und Extraktion eine gesamte Menge von 5.500 kg pro Stunde Frischwasser zugespeist.

Es werden pro Stunde 595 kg Extrakt zurück gewonnen. Aus dem Extraktor wird ein feuchtes Produkt entnommen, das fast extraktfrei ist, und noch ca. 10 % Wasser enthält.

## Patentansprüche

1. Kontinuierliches Verfahren zur Extraktion von Monomeren und Oligomeren des Caprolactams aus dem bei der Polymerisation zu Polyamid-6 erhaltenem Polymer-Rohprodukt, wobei eine Polymerschmelze in einen Granulator eingeführt wird und das Polyamid mit im Granulator im Kreislauf umlaufenden Wasser vor-extrahiert wird und anschließend das so erhaltene Rohgranulat in eine Extraktionskolonne überführt und mit Frischwasser **im Gegenstrom** behandelt wird, wobei sich in dem dabei anfallenden Extraktwasser Monomere und Oligomere des Caprolactams anreichern, **dadurch gekennzeichnet, dass** für die Granulation kein Frischwasser zugespeist wird, sondern ein Teilstrom des Extraktwassers aus der Extraktionskolonne mit einem Extraktgehalt von weniger als **1 Gew.-%** Monomeren und Oligomeren des Caprolactams kontinuierlich dem im Kreislauf umlaufenden Wasser des Granulators zugespeist wird, **bis dessen Extraktgehalt auf 4 - 6 % gestiegen ist** und wobei das angereicherte Prozesswasser aus der Granulation in die Extraktionskolonne rückgeführt wird.

2. Verfahren nach Anspruch 1, **dadurch gekennzeichnet, dass** das aus der Granulieranlage abgezogene **und mit Monomeren und Oligomeren angereicherte** Prozesswasser entweder verworfen oder erneut zur Extraktion eingesetzt oder in eine Rückgewinnungsanlage geleitet wird, um Wasser und Extrakt voneinander zu trennen.

## Claims

1. A continuous method for extracting monomers and oligomers of caprolactam from the polymer raw product obtained during polymerisation to form polyamide 6, a polymer melt being introduced into a granulator and the polyamide being pre-extracted with water circulating in the granulator, and the raw granulate thus obtained then being transferred into an extraction column and treated with fresh water in counterflow, monomers and oligomers of caprolactam becoming enriched in the extract water produced during this process, **characterised in that** no fresh water is fed for the granulation, but a partial stream of the extract water from the extraction column with an extract content of less than 1 % by weight of monomers and oligomers of caprolactam is fed continuously to the circulating water of the granulator until the extract content thereof has risen to 4-6 %, the enriched process water from the granulation being fed back into the extraction column.

2. The method according to Claim 1, **characterised in that** the process water removed from the granulating system and enriched with monomers and oligomers is either discarded or used again for extraction or is conveyed into a recovery system in order to separate water and extract from one another.

## Revendications

1. Procédé continu d'extraction de monomères et d'oligomères du caprolactame à partir du produit du polymère brut obtenu lors de la polymérisation en polyamide-6, lors duquel on introduit une masse fondue de polymère dans un granulateur et on pré-extrait le polyamide avec de l'eau tournant en circuit dans le granulateur et on transfère par la suite le granulat brut ainsi obtenu dans une colonne d'extraction et on le traite avec de l'eau fraîche à contre-courant, des monomères et des oligomères du caprolactame s'accumulant dans l'eau d'extraction produite, **caractérisé en ce que** pour la granulation, on ne rajoute pas d'eau fraîche, mais on rajoute un flux partiel de l'eau d'extraction provenant de la colonne d'extraction, avec une teneur en extrait inférieure à 1 % en poids de monomères et d'oligomères du caprolactame à l'eau tournant en circuit du granulateur, jusqu'à ce que sa teneur en extrait ait grimpé à de 4 à 6 % et l'eau de processus enrichie issue de la granulation étant recyclée dans la colonne d'extraction.

2. Procédé selon la revendication 1, **caractérisé en ce que** soit on jette ou on réutilise pour l'extraction l'eau soutirée de l'installation de granulation ou on la dirige vers une installation de récupération, pour séparer l'un de l'autre l'eau et l'extrait.
